Europäisches Patentamt

European Patent Office

Office européen des brevets

(11) Publication number: **0 265 163**
A2

# EUROPEAN PATENT APPLICATION

(21) Application number: 87309070.8

(22) Date of filing: 14.10.87

(51) Int. Cl.⁴: **A61M 16/10**

(30) Priority: 16.10.86 US 919673

(43) Date of publication of application:
27.04.88 Bulletin 88/17

(84) Designated Contracting States:
BE CH DE ES FR GB IT LI SE

(71) Applicant: INTERTECH RESOURCES INC.
2275 Half Day Road
Bannockburn Illinois 60015(US)

(72) Inventor: Wallace, Dean R.
1447 Tredegar Drive
Fort Myers Florida 33907(US)

(74) Representative: Newstead, Michael John et al
Page & Co. Temple Gate House Temple Gate
Bristol BS1 6PL(GB)

(54) **Heat and moisture exchanger.**

(57) This disclosure relates to a heat-moisture exchanger comprising first and second parts which are secured together to form a hollow housing. Ports are formed in the housing for connection to a patient and to a source of breathable gas, and the hollow interior of the housing forms a passageway for inhalation and exhalation gases to and from the patient. An insert is mounted within the housing and across the passageway, and comprises first and second materials. The first material forms a large surface area for the exchange of heat and moisture by condensation and evaporation. The second material is hydrophilic and absorbs moisture and then releases it by evaporation. The insert further acts as a filter of bacteria and virus.

FIG-3

## Field and Background of the Invention

This invention relates to a combination heat and moisture exchanger and filter for use in respiratory care and anesthesiology systems.

When a person is breathing in a normal manner through the nose or mouth, the inhaled air or other gas is warmed and humidified by the tissues of the mouth, nose and pharynx. By the time the gas reaches the bronchial tubes it is normally at body temeprature and at about 99% humidity.

However, when a patient is connected in a respiratory care system and a tracheal or tracheostomy tube is inserted into the trachea the gas is fed directly into the bronchial tubes. The gas thus bypasses the tissues and it is not adequately warmed and humidified. If the inhaled gas entering the bronchi is dry and cool, there will be a heat and moisture loss from the tracheal mucosa, causing it to dry and thicken and produce breathing problems.

Devices have been provided in the past to add heat and moisture to the inhaled gas. Humidifiers and vaporizors for this purpose are usually relatively expensive and require attention to the water supply and to the temperature and humidity controls.

An alternative to humidifiers and vaporizors as described above is a heat and moisture exchanger (HME). An exchanger contains an insert which removes heat and moisture from exhaled gas and then returns at least part of the heat and moisture to the subsequently inhaled gas. One type of exchanger collects moisture by condensation on the surfaces of the insert during exhalation and releases moisture by evaporization during inhalation.

A heat exchange process also occurs during operation of such an exchanger. Some heat is lost by convection through the walls of the tubes and the exchanger. Further, the heat of condensation is given up by the moisture to the walls of the exchanger (where some is lost), and the heat of vaporization is taken by the moisture from the walls.

In addition to the foregoing prior art, the following patents relate to breathing systems:

U.S.:

| 443,191 | 4,063,913 | British: |
|---------|-----------|----------|
| 3,491,754 | 4,133,656 | 2,053,694 |
| 3,814,094 | 4,171,962 | |
| 3,932,153 | 4,318,398 | |
| 3,941,862 | | |

Patent No. 443,191 shows an air filter including cotton/wool soaked with medicine or glycerine. No. 3,491,754 shows a breath warmer including layers of foam, metal foil and nylon mesh. No. 3,814,,094 shows a breath warmer including layers of aluminum screen and foam rubber. No. 3,932,153 shows a bacteria filter including a disk of porous Teflon impregnated with micro glass fiber on one side and nylon or glass cloth on the other side. The disk has its edge clamped between two housing parts. No. 3,941,862 shows a gas diffuser for use in a humidifier. No. 4,063,913, No. 4,133,656 and No. 4,171,962 show a bacteria filter including filter paper. No. 4,318,398 shows a humidity exchanger including vapor permeable fiber tubes. British No. 2,053,694 shows an exchanger including tubular fibers.

It is a general object of this invention to provide an improved heat and moisture exchanger which is also a bacterial and viral filter.

## Summary of the Invention

Apparatus in accordance with this invention comprises first and second housing parts which are secured together to form a hollow housing. Ports or openings are formed in the housing for connection to a patient and to a source of breathable gas, and the hollow interior of the housing forms a passageway for inhalation and exhalation gases flowing to and from the patient. An insert is mounted within the housing and across the passageway, and comprises first and second materials. The first material forms a large surface area for the exchange of heat and moisture by condensation and evaporation. The second material is hydrophilic and absorbs moisture and then releases it by evaporation. The insert further acts as a filter of bacteria and virus.

## Brief Description of the Drawings

The foregoing and other objects and advantages of the present invention will become apparent from the following detailed description taken in conjunction with the accompanying figures of the drawing, wherein:

Fig. 1 is an illustration of an exchanger in accordance with the invention in use by a patient;

Fig. 2 is an enlarged exploded view of the exchanger;

Fig. 3 is a sectional view of the exchanger;

Fig. 4 is a view taken on the line 4-4 of Fig. 2; and

Fig. 5 is a view taken on the line 5-5 of Fig. 2.

## Detailed Description of the Drawings

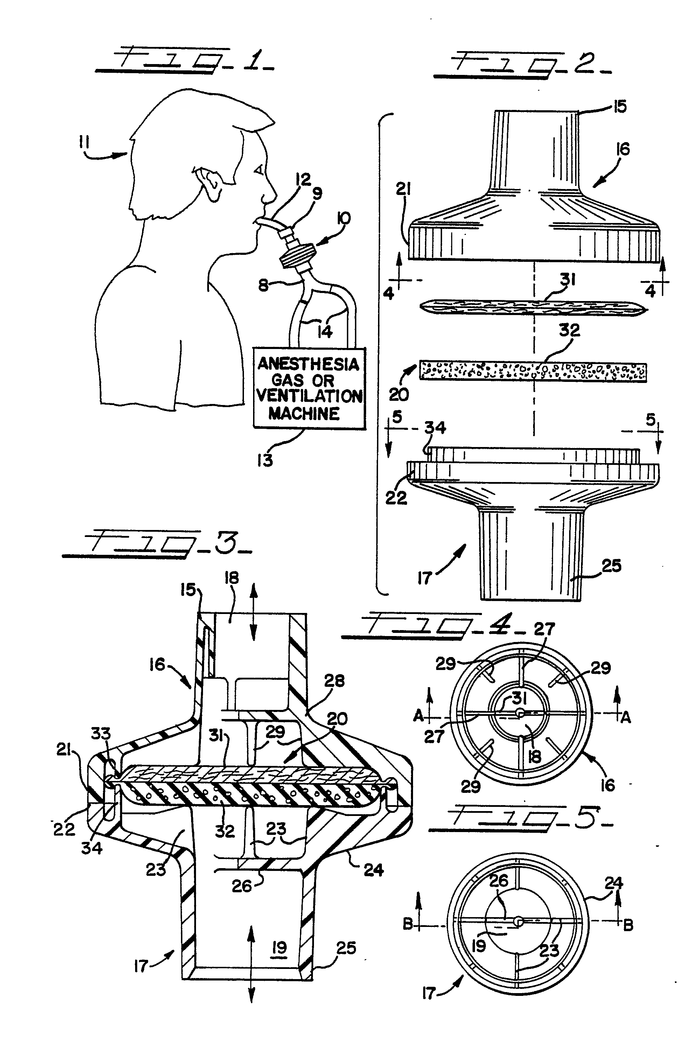

Fig. 1 shows a heat and moisture exchanger 10 in use by a patient indicated generally by the numeral 11. An endotracheal tube 12 is connected between an adaptor 9 and the patient, the tube 12 extending into the trachea of the patient, and the adaptor is connected to the exchanger. The other side of the exchanger 10 is connected to the center leg of a Y-piece 8, the other two legs of the Y-piece being connected by tubes 14 to a machine 13 such as an anesthesia gas or ventilation machine which is designed to supply a breathable gas to the patient 11. The gas may be oxygen or an anesthetic, for example. During such use, both the inhaled and the exhaled gases flow through the exchanger 10. During exhalation, a portion of the heat and the moisture of the exhaled gas is removed from the gas by the exchanger 10, and during inhalation, heat and moisture are transferred from the exchanger 10 to the gas prior to inhalation by the patient.

The heat and moisture exchanger 10 comprises first and second housing parts 16 and 17, which are generally funnel-shaped and have outer walls 28 and 24. The part 16 has an opening 18 formed at its small diameter end 15, this end 15 being adapted to be connected to the adaptor 9 and the endotracheal tube 12. Similarly, the other part 17 has an opening 19 formed in its small diameter end 25 which is adapted to be connected to the Y-piece 8 and the gas machine 13. The two units 16 and 17 further have large diameter ends 21 and 22, respectively, which are positioned against each other and secured together. The two housing parts may be formed of molded polyethylene and are secured together by such means as sonic welding. The housing parts 16 and 17 thus form a hollow interior which serves as a passageway for gas flowing between the two openings 18 and 19, and an insert 20 is mounted across the passageway.

With specific reference to Figs. 3 and 5, the section of the part 17 shown in Fig. 3 is taken on the line B-B of Fig. 5. The interior of the part 17 has four interior ribs which extend radially inwardly from the outer wall 24 to the diameter of the smaller opening 19, the ribs 23 being provided to strengthen the wall 24. In addition, a diagonally extending rib or brace 26 extends across the opening 19, which prevents a connecting tube from being inserted excessively far into the housing. The rib 26 also creates air turbulance within the housing and thereby increases the efficiency of the exchanger.

Similarly, the section of the part 16 shown in Fig. 3 is taken on the line A-A of Fig. 4. The housing part 16 includes a plurality of radially extending ribs 27 which extend from the wall 28 to the interior diameter of the opening 18. Intermediate shorter ribs 29 may also be formed on the interior of the wall 28. Another diagonal rib 31 may be formed on the interior of the housing part 16 and extend across the opening 18, the rib 31 having the same function as the rib 26.

The insert 20 is disk-shaped and is formed by two side-by-side layers 31 and 32. The two layers 31 and 32 have essentially the same outer diameter, and this diameter is slightly less than the diameters of the large ends 21 and 22 of the two housing parts. Circular pinch rings 33 and 34 are also formed at the large ends 21 and 22 and are spaced radially inwardly a short distance from the outermost edges of the housing parts. The circular pinch ring 33 of the part 16 is recessed axially inwardly from the lower edge of the large diameter end 21, whereas the pinch ring 34 of the other part 17 extends upwardly a short distance out of the large diameter open end 22. When the ends 21 and 22 are butted against each other as shown in Fig. 3, the adjacent ends of the two pinch rings 33 and 34 are spaced a short distance apart and the outer periphery of the insert 20 is located between these adjacent ends, and as shown in Fig. 3, the pinch rings 33 and 34 tightly grip the outer periphery of the insert 20 and hold it in place. In addition, the ribs 23 and 29 of the two housing parts are closely adjacent the opposite sides of the insert 20 and prevent excessive movement of the insert.

While a specific example of a material for the housing and a method of assembling the housing parts are described, it should be understood that the invention encompasses other lightweight housing materials and methods of assembly.

The layer 31 of the insert 20 is formed of natural polypropylene fibers having a close weave or mesh. The fibers are permanently electrostatically charged and thus also form a bacterial and viral filter. Such material is commercially available for this purpose, and a layer having a weight of approximately 200 grams per square meter is preferred. A heavier (and therefore thicker or more dense) material such as 300 grams per square meter may produce an excessive pressure drop for a given flow rate of gas. On the other hand a lighter material such as 100 grams per square meter may not have sufficient surface area. The other layer 32 is an open-cell urethane plastic foam which is pretreated to absorb moisture. A suitable hydrophilic foam is sold by the Scott Foam Company under the trademark Acquell, and this type of foam can absorb approximately 30 times its weight in moisture. The foam releases moisture by evaporation when dry gas from the machine 13 flows through it to the patient. It is preferred that foam having a thickness of approximately 1/4 inch be used, and such a layer produces virtually no pressure drop even though it absorbs a large amount of moisture.

To manufacture the exchanger shown in the drawings, the two housing parts 16 and 17 are formed and the two layers 31 and 32 are cut to size and positioned adjacent each other. In one mode of assembly, the housing part 16 is positioned with the large diameter end 21 facing upwardly and the two layers 31 and 32 are positioned within the open end 21. The outer periphery of the insert 20 is substantially equal to the interior diameter of the wall 28 at the large diameter end 21. As shown in Fig. 3, the diameter of the insert 20 is slightly greater than the diameters of the pinch rings 33 and 34, and the insert 20 is positioned with its outer periphery on the pinch ring 33. The other part 17 is then positioned over the open end 21 and the two pinch rings 33 and 34 pinch and grip the outer periphery of the insert. The ends 21 and 22 are then secured together by means such as an ultrasonic welding process, as previously mentioned.

In the use of the unit, the end 15 is connected to the adaptor and the endotracheal tube 12 and the end 25 is connected to the Y-piece 8. When the exhaled gas passes through the tubes and through the exchanger 10, the layers 31 and 32 collect moisture. The fiber layer 31 has a large surface area and moisture in the gas is condensed on the fiber surfaces. In addition, the treated foam layer 32 absorbs moisture as the exhaled gas passes through it. Some of the moisture condensed on the fibers is transferred to and absorbed by the foam layer 32 because of the close side-by-side relation of the layers 31 and 32, thereby increasing the effectiveness of both layers. Some of the moisture also condenses on the interior surfaces of the two housing parts 16 and 17. As the moisture condenses, the latent heat of condensation in the moisture is released and it warms the layers and the housing. When the patient inhales, the gas moves in the other direction through the housing and into the patient 11. The moisture in the two layers 31 and 32 is released and evaporated by the dry gas, and the gas is also warmed by the exchanger.

In addition, the fiber layer 31 serves as a filter and traps bacteria and virus (and other particles) both during inhalation and during exhalation.

It will be apparent from the foregoing that a novel and useful heat and moisture exchanger and filter has been provided. There are a number of factors that should be considered in the design of a heat and moisture exchanger. First, the exchanger should have sufficient size and capacity to add the needed amount of heat and moisture to the air flowing to the patient. However, the second and third factors limit the size of the exchanger. The second factor is that the exchanger should be small and lightweight because it is often supported by the patient on the tracheal tube. Third, its interior should be small because the dead space in the housing should be kept to a minimum. In the specific example illustrated and described herein, the diameter of the ends 21 and 22 is substantially 6.3 cm, and the total dead space within the housing is substantially 40 cc. Still another factor is that the pressure drop, or the resistance to air flow, across the exchanger should be as low as possible so that it does not hinder the patient's breathing.

The exchanger described herein meets these conflicting requirements. The two layers of the insert cooperate to exchange an adequate amount of heat and moisture even though they are relatively small in diameter and thin. There is little pressure drop across the foam layer and the fiber layer is thin enough that it does not significantly obstruct breathing. The parts of the exchanger are relatively small and are made of lightweight materials. Further, the ribs within the housing parts create gas turbulance which distributes the gas flow throughout the insert. In addition, the insert operates as a filter in addition to its other functions.

## Claims

1. A heat-moisture exchanger comprising a housing forming a hollow interior and having two flow openings formed therein, said interior and said openings forming a two directional gas flow passage through said housing between said openings, and an insert mounted in said housing and extending across said passage, said insert comprising a first layer of fibers forming a bacterial and viral filter, and a second layer of a hydrophilic material, each of said layers having substantially flat sides and positioned in close side-by-side relation with said flat sides extending across and perpendicular to said gas flow passage, said flat sides being in mutual contact, and all of said gas flowing through said insert in both directions of gas flow.

2. A heat-moisture exchanger according to Claim 1, wherein said first layer comprises of polypropylene fibers.

3. A heat-moisture exchanger according to Claim 2, wherein said second layer comprises an open-cell urethane plastic foam and means to enhance absorption of moisture.

4. A heat-moisture exchanger according to Claim 1, wherein said housing comprises first and second parts each having one of said flow openings therein, said first and second parts being secured together and clamping said insert between them.

5. A heat-moisture exchanger according to Claim 4, wherein said first and second parts of said housing have pinch rings formed thereon, and said insert is clamped between said pinch rings.

6. A heat-moisture exchanger according to Claim 1, wherein said housing has ribs formed therein, said ribs strengthening said housing and at least some of said ribs causing turbulance of gas flowing through said insert.

_FIG_1_

_FIG_2_

ANESTHESIA
GAS OR
VENTILATION
MACHINE

_FIG_3_

_FIG_4_

_FIG_5_